# EUROPÄISCHE PATENTSCHRIFT

(11) **EP 4 344 709 B1**
(45) Veröffentlichungstag und Bekanntmachung des Hinweises auf die Patenterteilung: **06.05.2026**
(21) Anmeldenummer: 23194490.1
(22) Anmeldetag: 31.08.2023
(51) Int. Cl.: A61L 2/18, A61B 90/40

(54) **VERFAHREN ZUR VALIDIERBAREN REINIGUNG VON SEMIKRITISCHEN ULTRASCHALLSONDEN**
METHOD FOR THE VALIDATABLE PURIFICATION OF SEMI-CRITICAL ULTRASOUND PROBES
PROCÉDÉ DE NETTOYAGE VALABLE DE SONDES ULTRASONORES SEMI-CRITIQUES

(30) Priorität: 09.09.2022 DE 102022123070
(43) Veröffentlichungstag der Anmeldung: 03.04.2024
(73) Patentinhaber: Dr. Schumacher GmbH, 34323 Malsfeld-Beiseförth (DE)
(72) Erfinder: Mäffert, Holger, Malsfeld (DE); Ballez, Mike, Malsfeld (DE); Kermanjani, Ali, Malsfeld (DE); Schumacher, Sönke, Malsfeld (DE); Michels, Winfried, Warburg (DE); Schneider, Franziska, Malsfeld (DE); Dittmar, Randolf, Malsfeld (DE); Birkelbach, Anke, Malsfeld (DE); Vaupel, Jonas, Malsfeld (DE)
(74) Vertreter: Sandvoß, Stefanie

(56) Entgegenhaltungen:
- WO-A1-2021/234214
- WO-A2-2012/141672
- CN-A- 113 082 241
- CN-A- 114 101 155
- CN-U- 215 613 590
- DE-U1- 202020 005 540
- NN: "Aufbereitung von Ultraschallsonden mit Schleimhautkontakt", 1 February 2019 (2019-02-01), pages 1 - 10, XP093124496, Retrieved from the Internet <URL:https://vah-online.de/files/download/vah-mitteilungen/VAH_Sonden_HM_1-2_19.pdf> [retrieved on 20240126]
- VON ULTRASCHALLSONDEN AUFBEREITUNG ET AL: "Empfehlung des Fachausschusses Qualität (101)", 12 June 2017 (2017-06-12), pages 1 - 3, XP093124452, Retrieved from the Internet <URL:https://www.dgsv-ev.de/wp-content/uploads/2016/09/AKQ_d_ZT_3_2017_Aufbereitung-von-Ultraschallsonden.pdf> [retrieved on 20240126]
- MÖLLERS MAREIKE ET AL: "Desinfektion von transvaginalen Ultraschallsonden - ein aktueller Überblick über Methoden und Empfehlungen", GYNAEKOLOGE, SPRINGER VERLAG, BERLIN, vol. 54, no. 9, 7 July 2021 (2021-07-07), pages 688 - 693, XP037559193, ISSN: 0017-5994, [retrieved on 20210707], DOI: 10.1007/S00129-021-04824-2
- NN: "Automatisierte Desinfektion: "Best-Practice" für semikritische Ultraschallsonden", 2 March 2022 (2022-03-02), pages 1 - 4, XP093124276, Retrieved from the Internet <URL:https://www.nanosonics.eu/media/qkab0ebg/mm01746-de-cb-v01-feb_22-automated-disinfection-best-practice-for-semi-critical-ultrasound.pdf> [retrieved on 20240126]
- NEUMANN GERD: "Hygienestandards in der gynäkologischen Praxis", GYNAEKOLOGE, SPRINGER VERLAG, BERLIN, vol. 54, no. 6, 7 May 2021 (2021-05-07), pages 419 - 427, XP037487361, ISSN: 0017-5994, [retrieved on 20210507], DOI: 10.1007/S00129-021-04803-7

## Beschreibung

Die vorliegende Erfindung betrifft ein Verfahren zur validierbaren Reinigung von semikritischen Ultraschallsonden, sowie ein zur validierbaren Reinigung von semikritischen Ultraschallsonden verwendbares Kit-of-parts.

In Fachkreisen wird schon seit längerem die Frage diskutiert, ob die gesetzlich geforderte Validierbarkeit der Aufbereitung von semikritischen Medizinprodukten, wie z.B. von Ultraschallsonden mit Schleimhautkontakt, mittels Wischdesinfektion vollumfänglich erfüllt werden kann. Vor diesem Hintergrund mutet es befremdlich an, dass zwar ausführlich über eine Validierbarkeit der Desinfektion von semikritischen Medizinprodukten diskutiert wird, während die der Desinfektion vorhergehende Reinigung dieser Medizinprodukte gar nicht erwähnt wird.

Dabei sollte es selbstverständlich sein, dass semikritische Medizinprodukte nach der Benutzung am Patienten gereinigt, und erst, wenn diese Reinigung unter Erfüllung der einschlägigen Anforderungen erfolgreich war, desinfiziert bzw. sterilisiert werden. Üblicherweise werden semikritische Ultraschallsonden mit einer Schutzhülle eingesetzt, in der sich ein Transmissionsgel befindet. Direkte Kontaminationen von Seiten des Patienten treten daher kaum auf. Insbesondere beim Abziehen der Schutzhülle nach einer erfolgten Untersuchung kann es jedoch zu Schmierkontaminationen kommen.

Da Ultraschallsonden nach der erfolgten Untersuchung insbesondere in Vertiefungen, z.B. für Biopsieaufsätze, stark mit Transmissionsgel verschmutzt sind, beeinträchtigt es die Desinfektion der Geräte, wenn das Transmissionsgel bei der der Desinfektion vorhergehenden Reinigung nur unzureichend entfernt wird. Überdies baut das zurückbleibende Gel mit der Zeit einen kaum zu entfernenden Rückstand auf, der zu Kontamination und Verfärbungen führt.

Das Gel besteht aus Carbomeren, wobei Rückstände des Gels mittels Bestimmung des Kohlenstoffgehalts pro Flächeneinheit nachgewiesen und mittels TOC (total organic carbon)-Messung quantifiziert werden können. Die DIN EN ISO 15883-5 enthält einen Warnwert von 6 µg Kohlenstoff/cm², der zur Beurteilung des Reinigungserfolgs herangezogen werden kann. Dieser Warnwert wird in der Praxis oftmals um ein Vielfaches überschritten, wie Prüfungen von semikritischen Ultraschallsonden, die mittels Wischtüchern gereinigt wurden, ergeben haben. Es ist daher davon auszugehen, dass die nachfolgende Desinfektion - unabhängig von der Desinfektionsmethode - durch die Rückstände beeinträchtigt wird und nicht sicher sein kann. Mit anderen Worten führt die mangelhafte Reinigung der Sonden trotz nachfolgender validierbarer Desinfektion dazu, dass der gesamte Aufbereitungsprozess als nicht validiert angesehen werden muss.

Ein solches aus dem Stand der Technik bekanntes und nicht validierbares Reinigungsverfahren semikritischer Ultraschallsonden wird zum Beispiel in B. Amann: "Aufbereitung von Ultraschallsonden", Zentralsterilisation 3/2017, S. 195-197 beschrieben. Unmittelbar nach der Anwendung der Sonde werden hier die verwendete Einwegschutzhülle und das Ultraschallgel entfernt und anhaftende Verschmutzungen werden mittels feuchtem Einwegtuch entfernt. Anschließend folgt eine nicht näher beschriebene Desinfektion, die durch geeignete Geräte automatisiert bzw. übernommen werden kann. Nachteilhaft ist hier also, dass die der Desinfektion vorhergehende Reinigung nur durch ein manuelles Abwischen des Ultraschallgels mit einem Tuch erfolgt, wobei dies keine Validierbarkeit erlaubt.

Auch in der Arbeitsanleitung "Aufbereitung von Ultraschallsonden für transvaginale und abdominale Untersuchungen", Dr. Schumacher GmbH, Stand 06/2017 ("https://www.schumacher-online.com/fileadmin/Docs/Printmedia/Instructions/DrS_Anleitung _Ultraschallsonden_A3_DE_WEB.pdf") wird zur Reinigung von Sonden beschrieben, dass diese nach dem Entfernen der Schutzhülle mit einem zuvor mit Wasser befeuchteten Tuch vorgereinigt werden sollen. Nach einem weiteren Reinigungsschritt mit einem weiteren Tuch erfolgt die Desinfektion als Wischdesinfektion ebenfalls mit einem Tuch, anschließend wird die Sonde getrocknet. Auch hier wird also lediglich eine nicht validierbare Reinigung durch Abwischen der Sonde mit Tüchern beschrieben.

Die Präsentation "Erstellung einer Anleitung für die Aufbereitung von Ultraschallsonden mittels Wischdesinfektion", DGKH-Kongress 2016 ("https://www.krankenhaushygiene.de/ referate/1d057088c0e892e9c2fd88afed9b6d6f.pd) offenbart einzelne Schritte zum Reinigen von Ultraschallsonden, insbesondere ein Abwischen der Sonde nach dem Entfernen der Schutzhülle mit einem mit Wasser befeuchteten Tuch, eine manuelle Reinigung mit einem mit Desinfektionsmittel getränkten Tuch und eine Trocknung der Sonde. Auch hier erfolgt die der Desinfektion vorhergehende Reinigung also nur durch nicht validierbares Abwischen der Sonde mit Tüchern.

### Aufgabe der Erfindung

Es ist die Aufgabe der vorliegenden Erfindung, ein alternatives Verfahren zur Reinigung von semikritischen Ultraschallsonden bereitzustellen, das die vorgenannten aus dem Stand der Technik bekannten Nachteile vorzugsweise überwindet und sich insbesondere durch eine objektive Validierbarkeit auszeichnet.

Eine weitere Aufgabe der vorliegenden Erfindung ist es, ein Verfahren zur Reinigung von semikritischen Ultraschallsonden bereitzustellen, nach dessen Durchführung der Warnwert von 6 µg Kohlenstoff/cm² gemäß DIN EN ISO 15883-5 auf der Oberfläche der gereinigten Geräte unterschritten wird.

In "Aufbereitung von Ultraschallsonden mit Schleimhautkontakt", 1. Februar 2019 (2019-02-01), Seiten 1-10, XP093124496, gefunden im Internet am 26.01.2024 unter URL:https://vah-online.de/files/download/vah-mitteilungen/VAH_Sonden_HM_1-2_19.pdf beschreibt, dass zur Aufbereitung transvaginaler Ultraschallsonden Gelreste nach dem Entfernen der Schutzhülle mit einem trockenen Tuch entfernt werden. Erst anschließend wird mit einem feuchten Desinfektionstuch nachgereinigt. Im Zusammenhang mit TEE-Sonden wird das Einführen der Ultraschallsonde in eine Vorrichtung zur Reinigung von Ultraschallsonden beschrieben, die eine die Sonde kontaktierende Elutionslösung enthält.

In "Aufbereitung von Ultraschallsonden", Empfehlung des Fachausschusses Qualität (101), 12. Juni 2017 (2017-06-12), Seiten 1-3, XP093124452, gefunden im Internet am 26.01.2024 unter URL:https://www.dgsv-ev.de/wp-content/uploads/20 16/09/AKQ_d_ZT _3_2017 _Aufbereitung-von-Ultraschallsonden.pdf wird bezüglich der Reinigung von semikritischen Ultraschallsonden beschrieben, dass unmittelbar nach der Anwendung der Sonde die Einwegschutzhülle und das Ultraschallgel zu entfernen und anhaftende Verschmutzungen mittels feuchtem Einwegtuch zu entfernen sind. Anschließend folgt eine nicht näher beschriebene Desinfektion, die durch geeignete Geräte automatisiert bzw. übernommen werden kann.

In "Desinfektion von transvaginalen Ultraschallsonden - ein aktueller Überblick über Methoden und Empfehlungen" von Möllers et al., Gynaekologe, Springer Verlag, Berlin, Bd. 54, Nr. 9, 7. Juli 2021 (2021-07-07), Seiten 688-693, XP037559193, ISSN:0017-5994, DOI: 10.1007/S00129-021-04824-2 wird offenbart, dass eine maschinelle Reinigung/Desinfektion von semikritischen Ultraschallsonden einer Wischdesinfektion vorzuziehen ist, da letztere nicht validierbar sei.

Die WO 2012/141672 A2 beschreibt ein Reinigungsbehältnis für Ultraschallsonden, die für Untersuchungen am Auge verwendet werden. Das Reinigungsbehältnis ist dabei vorzugsweise ein Beutel mit Reinigungsflüssigkeit, in den das distale Ende der Sonde eingetaucht wird und dann die Öffnung abgedichtet wird.

Die CN 113082241A beschreibt eine Vorrichtung und ein Verfahren zum Sterilisieren von transvaginalen Ultraschallsonden. Dabei werden die Sonden zunächst mit Chlordioxid-Tüchern vordesinfiziert und anschließend mit UV-Licht sterilisiert.

### Allgemeine Beschreibung der Erfindung

Die Erfindung löst diese Aufgabe mit den Merkmalen der Ansprüche und insbesondere mit einem Verfahren zur Reinigung von semikritischen Ultraschallsonden, das die folgenden Schritte umfasst oder daraus besteht:
a) Abziehen einer Schutzhülle von der Ultraschallsonde;
b) Entfernung von an der Ultraschallsonde haftendem Transmissionsgel mittels eines mit einem Tränkungsmittel getränkten Tuches;
c) Einführen der Ultraschallsonde in eine Vorrichtung zur Reinigung von Ultraschallsonden, wobei die Vorrichtung zur Reinigung von Ultraschallsonden eine Elutionslösung enthält, die die Ultraschallsonde kontaktiert; und
d) Entfernen der Ultraschallsonde aus der Vorrichtung zur Reinigung von Ultraschallsonden und Trocknen der Ultraschallsonde.

Im Stand der Technik werden Ultraschallsonden bislang häufig unzulänglich gereinigt, indem die Geräte nach dem Entfernen der Schutzhülle von der Ultraschallsonde zunächst mit einem trockenen Tuch von anhaftendem Transmissionsgel befreit werden und anschließend mit einem feuchten Tuch nachgewischt wird, bevor die Sonden desinfiziert werden.

Nachdem sich diese Art der Reinigung als unzureichend herausgestellt hat, wurde nun überraschend gefunden, dass eine direkte Reinigung mit einem feuchten Tuch, d.h. mit einem Vliesstoff, der mit einem Tränkungsmittel getränkt wurde, gefolgt von einer Reinigung mittels Elutionslösung nicht nur zu signifikant besseren, sondern auch zu validierbaren Reinigungsergebnissen führt.

Vorzugsweise werden dabei für den Schritt der Entfernung von an der Ultraschallsonde haftendem Transmissionsgel nacheinander zwei mit einem Tränkungsmittel getränkte Tücher verwendet. Diese Tücher können beispielsweise Viskosefasern, Lyocell-Fasern, PET-Fasern, PP-Fasern, Hanffasern, Cellulosefasern, Flachsfasern, Mikrofasern, Baumwollfasern und Mischungen dieser umfassen oder daraus bestehen. Das Tränkungsmittel, mit dem die Tücher imprägniert sind, ist dabei vorzugsweise ein Desinfektionsmittel oder alternativ ein wasserbasiertes Tränkungsmittel mit einem Konservierungsmittel bzw. Mikrobizid.

Selbstverständlich können auch mehr als zwei mit einem Tränkungsmittel getränkte Tücher zur Entfernung des Transmissionsgels verwendet werden, dies ist aber in der Regel nicht notwendig.

Unter "Ultraschallsonden" werden im Rahmen der vorliegenden Erfindung semikritische Ultraschallsonden verstanden, insbesondere transvaginale und transrektale Ultraschallsonden.

Nach dem Schritt der Entfernung von an der Ultraschallsonde haftendem Transmissionsgel mittels eines mit einem Tränkungsmittel getränkten Tuches oder mittels zwei mit einem Tränkungsmittel getränkten Tüchern werden bevorzugt mit einem Hilfswerkzeug sichtbare Reste von Transmissionsgel aus Vertiefungen oder Einkerbungen in der Oberfläche der Ultraschall-sonde entfernt, die z.B. für Biopsieaufsätze vorhanden sind und in denen sich das Transmissionsgel während der Anwendung vermehrt sammelt. Ein dazu geeignetes Hilfswerkzeug ist insbesondere stäbchenförmig und weist ein weiches Ende, beispielsweise aus Stoff, Watte oder Silikon auf. Ein besonders geeignetes und kostengünstiges Hilfswerkzeug ist ein Wattestäbchen, insbesondere ein trockenes und/oder doppelendiges Wattestäbchen.

Obwohl der Schritt des gezielten Entfernens von Transmissionsgel-Resten aus Vertiefungen der Ultraschallsonden mit einem Hilfswerkzeug nur optional ist, so ist dessen Durchführung doch stark bevorzugt, da dies das Reinigungsergebnis signifikant verbessert, wie an späterer Stelle noch beschrieben wird. Doch auch ohne eine gezielte Reinigung der Vertiefungen mit einem Hilfswerkzeug ist eine validierbare Reinigung mit dem erfindungsgemäßen Verfahren möglich.

Nach der Entfernung des Transmissionsgels mittels getränkter Tücher und optional mittels eines Wattestäbchens oder eines vergleichbaren Hilfswerkzeugs wird die Ultraschallsonde erfindungsgemäß in eine Vorrichtung zur Reinigung von Ultraschallsonden eingeführt, wobei die Vorrichtung zur Reinigung von Ultraschallsonden eine Elutionslösung enthält, die die Ultraschallsonde kontaktiert, insbesondere für mindestens 30 Sekunden und vorzugsweise für 30-60 Sekunden. Während dieser Zeit der Kontaktierung der Sonde mit der Elutionslösung sollte vorzugsweise die Ultraschallsonde und/oder die Vorrichtung zur Reinigung von Ultraschallsonden bewegt werden, sodass es zu einer Bewegung der Elutionslösung in der Vorrichtung zur Reinigung von Ultraschallsonden kommt, idealerweise sogar zu Spritzeffekten, durch welche die Elutionslösung besonders gut in die Vertiefungen der Sonde gelangt.

Bei der Elutionslösung handelt es sich vorzugsweise um eine Lösung mit einem pH-Wert im Bereich von 2-10, besonders bevorzugt um eine saure Lösung mit einem pH-Wert von 2-5 oder 2-4. Schon ein Volumen von 15 mL Elutionslösung kann für eine effektive Reinigung vollkommen ausreichend sein, sodass die in dem erfindungsgemäßen Verfahren verwendete Vorrichtung zur Reinigung von Ultraschallsonden vorzugsweise 15 mL saure Elutionslösung enthält. Als besonders geeignete Elutionslösungen haben sich eine Lösung mit 0,1% Peressigsäure mit einem pH-Wert von 4 sowie eine Lösung, die unter dem Handelsnamen "Manushield 0,5%" bekannt ist, einen pH-Wert von 8 aufweist und neben Wasser noch 10-25 Gew.-% ethoxylierte, verzweigte C9-C11-Alkohole, 1-5 Gew.-% denaturierten Alkohol, 1-5 Gew.-% C12-C15 Pareth-12, 0,1-1 Gew.-% Didecylmethylpoly(oxyethyl)ammonium-propionat, 0,1-1 Gew.-% Natriumcarbonat, 0,1-1 Gew.-% Dinatriumhydroxyethylaminobismethylphosphonat, 0-0,1 Gew.-% Glycol und 0-0,1 Gew.-% Zitronensäure enthält, herausgestellt.

In einer bevorzugten Ausführungsform des erfindungsgemäßen Verfahrens zur Reinigung von semikritischen Ultraschallsonden wird die Ultraschallsonde in eine Vorrichtung zur Reinigung von Ultraschallsonden eingeführt, die ein einseitig offenes Behältnis mit oder aus Kunststoff ist, in dem sich die Elutionslösung befindet. Gemäß einer besonders bevorzugten Ausführungsform handelt es sich bei dem einseitig offenen Behältnis mit oder aus Kunststoff dabei um einen Beutel, in den die Ultraschallsonde mit dem Ultraschallkopf voran eingeführt wird, wobei der Beutel nach dem Einführen der Ultraschallsonde um den Umfang der Ultraschallsonde herum manuell oder auf andere Weise abgedichtet bzw. verschlossen wird und für mindestens 30 Sekunden, vorzugsweise für 30-60 Sekunden, bewegt bzw. geschüttelt wird. Insbesondere durch Schütteln kommt es dabei zu Spritzeffekten der Elutionslösung, die für die Reinigung der Sonde, insbesondere von deren Vertiefungen äußerst vorteilhaft sind.

In einer alternativen Ausführungsform wird als einseitig offenes Behältnis mit oder aus Kunststoff ein Becher eingesetzt, auf dessen innenliegender Oberfläche flexible Elemente angeordnet sind, wobei die Ultraschallsonde mit dem Ultraschallkopf voran in den Becher eingeführt wird und für mindestens 30 Sekunden, vorzugsweise für 30-60 Sekunden, manuell oder auf andere Weise in dem Becher bewegt wird. Bei den flexiblen Elementen kann es sich beispielsweise um Noppen, z.B. aus Silikon, oder Borsten, z.B. aus Nylon, handeln. Bei der Bewegung der Ultraschallsonde in dem Becher, die auf- und abwärts gerichtete Bewegungen sowie Drehbewegungen beinhalten kann, erfolgt mit Hilfe der flexiblen Elemente, die zusammen mit der Elutionslösung die Oberfläche der Sonde kontaktieren, eine gründliche Reinigung auch in den Vertiefungen der Sonde.

In einer weiteren alternativen Ausführungsform des erfindungsgemäßen Verfahrens wird die zu reinigende semikritische Ultraschallsonde in eine Vorrichtung zur Reinigung von Ultraschallsonden eingeführt, die eine Folie mit daran angeordnetem saugfähigem Material ist. Das saugfähige Material ist dabei mit einer Elutionslösung getränkt, wobei die Folie mit dem daran angeordneten saugfähigen Material mit dem mit Elutionslösung getränkten saugfähigen Material die Ultraschallsonde kontaktiert und für mindestens 30 Sekunden, vorzugsweise für 30-60 Sekunden, auf der Oberfläche der Ultraschallsonde bewegt wird.

Unabhängig von der gewählten Vorrichtung zur Reinigung von Ultraschallsonden erfolgt der Schritt des Kontaktierens der Ultraschallsonde mit der Elutionslösung bevorzugt für 30-60 Sekunden. In Ausführungsformen des erfindungsgemäßen Verfahrens, in denen nach der Entfernung von an der Ultraschallsonde haftendem Transmissionsgel mittels eines oder mehrerer mit einem Tränkungsmittel getränkten Tuches/Tücher noch eine Entfernung von Transmissionsgelresten aus Vertiefungen in der Oberfläche der Ultraschallsonde mit einem Hilfswerkzeug erfolgt, ist es bereits ausreichend, die Ultraschallsonde mit der Elutionslösung für 30 bis 35 Sekunden zu kontaktieren, während es bei Verzicht auf diesen optionalen Schritt des Entfernens von Rückständen aus den Vertiefungen der Sonde mit einem Hilfswerkzeug bevorzugt ist, die Ultraschallsonde mit der Elutionslösung für 45 bis 60 Sekunden zu kontaktieren.

Nach der Kontaktierung der Ultraschallsonde mit der Elutionslösung wird die Ultraschallsonde aus der Vorrichtung zur Reinigung von Ultraschallsonden wieder entfernt und getrocknet, bevor die Desinfektion durchgeführt wird. Das Trocknen der Sonde kann dabei auf beliebige Weise erfolgen, z.B. mittels Abtrocknens bzw. Abwischens mit einem trockenen Tuch, mittels Trocknen an der Luft oder mittels Druckluft.

Weiterhin betrifft die vorliegende Erfindung auch ein Kit-of-parts, das Folgendes umfasst oder daraus besteht:
a) mit einem Tränkungsmittel getränkte Tücher; und
b) eine Elutionsmittel enthaltende Vorrichtung zur Reinigung von semikritischen Ultraschallsonden, wobei die Vorrichtung zur Reinigung von Ultraschallsonden ausgewählt ist aus einem Elutionslösung enthaltenden Behältnis und einer Folie mit daran angeordnetem saugfähigem Material, das mit Elutionslösung getränkt ist.

Die mit einem Tränkungsmittel getränkten Tücher weisen dabei die gleichen Merkmale auf wie im Voranstehenden in Bezug auf das erfindungsgemäße Verfahren beschrieben, insbesondere hinsichtlich der Zusammensetzung des Tränkungsmittels und des Vliesstoffes.

Auch die in dem Kit-of-parts enthaltene Elutionsmittel enthaltende Vorrichtung zur Reinigung von Ultraschallsonden weist die gleichen Merkmale auf wie im Voranstehenden in Bezug auf das erfindungsgemäße Verfahren beschrieben.

### Genaue Beschreibung der Erfindung

Die Erfindung wird nun genauer anhand von Beispielen beschrieben.

### Beispiel 1: Durchführung des erfindungsgemäßen Verfahrens zur validierbaren Reinigung von semikritischen Ultraschallsonden in einer ersten Ausführungsform

Eine semikritische Ultraschallsonde (Transvaginalsonde) mit einer Oberfläche von 260 cm² wurde mit einer Schutzhülle versehen und mit Transmissionsgel angeschmutzt, wobei darauf geachtet wurde, dass die Vertiefungen komplett mit dem Gel gefüllt waren. Anschließend wurde die Schutzhülle von der Ultraschallsonde abgezogen, indem die Hülle mit Daumen und Zeigefinger umfasst und nach distal abgeschoben wurde, wodurch die an der Ultraschallsonde verbleibende Gelmenge minimiert wurde. Die Ultraschallsonde wurde nun einmal mit einem herkömmlichen Feuchttuch zur Babyreinigung und -pflege ("Hipp Ultrasensitiv", Feuchttuch 1) von proximal nach distal abgewischt und das Feuchttuch anschließend entsorgt. Mit einem weiteren Feuchttuch derselben Beschaffenheit wurde daraufhin der Gel-behaftete Bereich der Ultraschallsonde erneut abgewischt. Anschließend wurden die Vertiefungen der Sonde mit einem doppelendigen Wattestäbchen ausgewischt, bevor die Ultraschallsonde in einen Folienschlauchbeute mit 15 mL Manushield-Lösung (0,1 %, pH 8) gegeben und für 30-35 Sekunden geschüttelt wurde. Nach dem Kontaktieren mit der Elutionslösung wurde die Ultraschallsonde mit Einweg-Küchentüchern getrocknet.

Der Reinigungserfolg des vorbeschriebenen Verfahrens wurde mittels Carbomerquantifizierung bzw. Ermittlung des TOC-Wertes überprüft.

Dieser Versuch wurde für eine höhere Aussagekraft der Ergebnisse viermal wiederholt und weitere fünf Mal auf identische Weise, jedoch unter Verwendung anderer herkömmlicher Feuchttücher zur Babyreinigung und -pflege ("Babydream" Feuchttuch 2) durchgeführt. Die ermittelten TOC-Werte sind in der nachstehenden Tabelle 1 angegeben.

**Tabelle 1: Ermittelte TOC-Werte nach Durchführung des erfindungsgemäßen Verfahrens unter Verwendung von Feuchttuch 1 und Feuchttuch 2**

| | TOC-Werte / µg C | |
|---|---|---|
| | Feuchttuch 1 | Feuchttuch 2 |
| Versuch 1 | 173,7 | 100,5 |
| Versuch 2 | 93,9 | 300,1 |
| Versuch 3 | 200,3 | 253,5 |
| Versuch 4 | 133,8 | 180,3 |
| Versuch 5 | 153,7 | 286,8 |

Die ermittelten TOC-Werte mit einem maximalen TOC-Wert von 300,1 µg C pro 260 cm² Sondenoberfläche zeigen, dass das erfindungsgemäße Verfahren zu hervorragenden und validierbaren Reinigungsergebnissen führt. Der Warnwert von 6 µg Kohlenstoff/cm², der in der Praxis bei aus dem Stand der Technik bekannten Reinigungsverfahren für semikritische Ultraschallsonden regelmäßig überschritten wird, wird in dem erfindungsgemäßen Verfahren sehr deutlich und zuverlässig unterschritten, denn bezogen auf die Reinigungsoberfläche der verwendeten Sonde von 260 cm² lag der durchschnittliche Kohlenstoffgehalt nach der vorstehend beschriebenen Reinigung bei 0,36 bis 1,15 µg Kohlenstoff/cm², was nur noch auf äußerst geringe Carbomer-Rückstände schließen lässt.

### Beispiel 2: Durchführung des erfindungsgemäßen Verfahrens zur validierbaren Reinigung von semikritischen Ultraschallsonden in einer zweiten Ausführungsform

Nach den gemäß Beispiel 1 erzielten hervorragenden Reinigungserfolgen sollte in diesem Beispiel ein Reinigungsverfahren getestet werden, das eine andere Elutionslösung einsetzt sowie auf eine Entfernung der Reste von Transmissionsgel mit einem Hilfswerkzeug aus den Vertiefungen der Ultraschallsonde verzichtet.

Analog zu der in Beispiel 1 beschriebenen ersten Ausführungsform des Verfahrens wurde auch bei diesem Verfahren jeder Schritt wie beschrieben durchgeführt, es wurde lediglich auf das Auswischen der Vertiefungen der Ultraschallsonde mit dem Reinigungswerkzeug (Wattestäbchen) verzichtet, und es wurden anstelle von 15 mL Manushield-Lösung (0,1 %, pH 8) 15 mL Peressigsäure (0,1 %, pH 4) als Elutionslösung verwendet. Als zusätzliche Abweichung wurde die Behandlung mit der Elutionslösung für 50-60 Sekunden durchgeführt.

Da der Reinigungserfolg mit Feuchttuch 1, wie in Beispiel 1 gezeigt wurde, vergleichbar mit dem Reinigungserfolg mit Feuchttuch 2 ist, wurde sich hier auf die Verwendung von Feuchttuch 1 beschränkt.

Die Experimente wurden fünf Mal durchgeführt, bevor jeweils die TOC-Werte der gereinigten Sonden bestimmt wurden. Die ermittelten TOC-Werte sind in der nachstehenden Tabelle 2 angegeben.

**Tabelle 2: Ermittelte TOC-Werte nach Durchführung des erfindungsgemäßen Verfahrens unter Verwendung von Peressigsäure als Elutionslösung und unter Verzicht auf eine Reinigung der Vertiefungen der Sonde mit Hilfswerkzeug**

| | TOC-Werte / µg C |
|---|---|
| Versuch 1 | 147,1 |
| Versuch 2 | 153,7 |
| Versuch 3 | 951,8 |
| Versuch 4 | 273,5 |
| Versuch 5 | 246,8 |

Die ermittelten TOC-Werte mit einem maximalen TOC-Wert von 951,8 µg C pro 260 cm² Sondenoberfläche zeigen, dass das erfindungsgemäße Verfahren auch mit Peressigsäure als Elutionslösung und sogar ohne eine Reinigung der Vertiefungen der Sonde mit einem Hilfswerkzeug zu validierbaren Reinigungsergebnissen führt. Bezogen auf die Reinigungsoberfläche der verwendeten Sonde von 260 cm² lag der Kohlenstoffgehalt nach der vorstehend beschriebenen Reinigung in allen Fällen bei 0,57 bis 3,66 µg Kohlenstoff/cm². Damit ist die Streuung der Ergebnisse zwar größer als bei Durchführung der zusätzlichen Reinigung der Vertiefungen mit einem Hilfswerkzeug, der Warnwert von 6 µg Kohlenstoff/cm² wird allerdings auch in dieser Ausführungsform des erfindungsgemäßen Verfahrens zuverlässig unterschritten.

Generell beträgt der TOC-Wert semikritischer Ultraschallsonden nach Durchführung des erfindungsgemäßen Verfahrens < 4 µg Kohlenstoff/cm², was belegt, dass sich das erfindungsgemäße Verfahren in seiner Reinigungswirkung signifikant von aus dem Stand der Technik bekannten Verfahren, die einen so geringen Kohlenstoffgehalt nicht erzielen können, unterscheidet.

Die in der vorstehenden Beschreibung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Verfahren zur validierbaren Reinigung von semikritischen Ultraschallsonden, umfassend die folgenden Schritte:
a) Abziehen einer Schutzhülle von einer Ultraschallsonde;
b) Entfernung von an der Ultraschallsonde haftendem Transmissionsgel mittels eines mit einem Tränkungsmittel getränkten Tuches;
c) Einführen der Ultraschallsonde in eine Vorrichtung zur Reinigung von Ultraschallsonden, wobei die Vorrichtung zur Reinigung von Ultraschallsonden eine Elutionslösung enthält, die die Ultraschallsonde kontaktiert; und
d) Entfernen der Ultraschallsonde aus der Vorrichtung zur Reinigung von Ultraschallsonden und Trocknen der Ultraschallsonde;
e) Durchführung einer Desinfektion.

2. Verfahren nach Anspruch 1, **dadurch gekennzeichnet, dass** für den Schritt der Entfernung von an der Ultraschallsonde haftendem Transmissionsgel nacheinander zwei mit einem Tränkungsmittel getränkte Tücher verwendet werden.

3. Verfahren nach Anspruch 1 oder 2, **dadurch gekennzeichnet, dass** nach dem Schritt der Entfernung von an der Ultraschallsonde haftendem Transmissionsgel mittels eines oder mehrerer mit einem Tränkungsmittel getränkten Tuches/Tücher mit einem Hilfswerkzeug Reste von Transmissionsgel aus Vertiefungen in der Oberfläche der Ultraschallsonde entfernt werden.

4. Verfahren nach einem der voranstehenden Ansprüche, **dadurch gekennzeichnet, dass** die Ultraschallsonde in eine Vorrichtung zur Reinigung von Ultraschallsonden eingeführt wird, die ein einseitig offenes Behältnis mit oder aus Kunststoff ist, in dem sich die Elutionslösung befindet.

## Claims

1. Process for the validatable cleaning of semi-critical ultrasound probes, comprising the following steps:
a) Removing a protective cover from an ultrasound probe;
b) Removing transmission gel adhering to the ultrasound probe using a cloth impregnated with an impregnating agent;
c) Inserting the ultrasound probe into a device for cleaning ultrasound probes, wherein the device for cleaning ultrasound probes contains an elution solution that is in contact with the ultrasound probe; and
d) Removing the ultrasound probe from the device for cleaning ultrasound probes and drying the ultrasound probe;
e) performing a disinfection.

2. Process according to claim 1, **characterized in that**, for the step of removing transmission gel adhering to the ultrasound probe, two cloths impregnated with an impregnating agent are used successively.

3. Process according to claim 1 or 2, **characterized in that**, following the step of removing transmission gel adhering to the ultrasound probe using one or more cloths impregnated with an impregnating agent, residues of transmission gel are removed from recesses in the surface of the ultrasound probe using an auxiliary tool.

4. Process according to one of the preceding claims, **characterized in that** the ultrasound probe is inserted into a device for cleaning ultrasound probes which is a container open on one side, made of or comprising plastic, in which the elution solution is contained.

## Revendications

1. Procédé pour le nettoyage validable de sondes semi-critiques à ultrasons, comprenant les étapes suivantes :
a) enlèvement d'une housse de protection d'une sonde à ultrasons ;
b) élimination de gel de transmission adhérant à la sonde à ultrasons au moyen d'un chiffon imprégné avec un produit d'imprégnation ;
c) introduction de la sonde à ultrasons dans un dispositif destiné au nettoyage de sondes à ultrasons, le dispositif destiné au nettoyage de sondes à ultrasons contenant une solution d'élution qui entre en contact avec la sonde à ultrasons ; et
d) enlèvement de la sonde à ultrasons hors du dispositif destiné au nettoyage de sondes à ultrasons et séchage de la sonde à ultrasons ;
e) exécution d'une désinfection.

2. Procédé selon la revendication 1, **caractérisé en ce que**, pour l'étape de l'élimination de gel de transmission adhérant à la sonde à ultrasons deux chiffons imprégnés avec un agent d'imprégnation sont utilisés successivement.

3. Procédé selon la revendication 1 ou 2, **caractérisé en ce qu'**après l'étape de l'élimination de gel de transmission adhérant à la sonde à ultrasons au moyen d'un ou de plusieurs chiffon(s) imprégné(s) avec un agent d'imprégnation, des résidus de gel de transmission sont éliminés de cavités dans la surface de la sonde à ultrasons à l'aide d'un outil auxiliaire.

4. Procédé selon l'une quelconque des revendications précédentes, **caractérisé en ce que** la sonde à ultrasons est introduite dans un dispositif destiné au nettoyage de sondes à ultrasons, qui est un récipient ouvert d'un côté, comportant de la matière plastique ou constitué de matière plastique, dans lequel se trouve la solution d'élution.
